# FASCICULE DE BREVET EUROPEEN

(11) **EP 3 714 661 B1**
(45) Date de publication et mention de la délivrance du brevet: **16.11.2022**
(21) Numéro de dépôt: 18762368.1
(22) Date de dépôt: 03.08.2018
(51) Int. Cl.: H05B 45/37, H05B 45/10

(54) **DISPOSITIF D'ECLAIRAGE FACILITANT LA LECTURE**
BELEUCHTUNGSVORRICHTUNG, DIE DAS LESEN ERLEICHTERT
LIGHTING DEVICE THAT FACILITATES READING

(30) Priorité: 20.11.2017 FR 1701200
(43) Date de publication de la demande: 30.09.2020
(60) Demande divisionnaire: 22200459.0
(73) Titulaire: Le Floch, Albert, 35700 Rennes (FR); Université de Rennes 1, 35000 Rennes (FR)
(72) Inventeur: LE FLOCH, Albert, 35700 Rennes (FR); ROPARS, Guy, 35000 Rennes (FR)
(74) Mandataire: Ex Materia
(86) Numéro de dépôt international: PCT/FR2018/052012
(87) Numéro de publication internationale: WO 2019/097128

(56) Documents cités:
- US-A1- 2013 021 386
- US-A1- 2013 021 386
- ALBERT LE FLOCH ET AL: "Left-right asymmetry of the Maxwell spot centroids in adults without and with dyslexia", BIOLOGICAL SCIENCES, vol. 284, no. 1865, 25 octobre 2017 (2017-10-25), page 20171380, XP055490044, ISSN: 0962-8452, DOI: 10.1098/rspb.2017.1380
- La vraie démocratie: "Des physiciens de Rennes ont percé le mystère de la dyslexie", Youtube, 19 octobre 2017 (2017-10-19), page 2 pp., XP054978716, Extrait de l'Internet: URL:https://www.youtube.com/watch?v=12EoF5 YS8Zg [extrait le 2018-09-27]
- Albert Le Floch ET AL: "Left-right asymmetry of the Maxwell spot centroids in adults without and with dyslexia Eleczronic Supplement Material", , 18 octobre 2017 (2017-10-18), pages 2-3, XP055510300, Extrait de l'Internet: URL:http://rspb.royalsocietypublishing.org /highwire/filestream/77828/field_highwire_ adjunct_files/0/rspb20171380supp1.pdf [extrait le 2018-09-26]
- Albert Le Floch ET AL: "Left-right asymmetry of the Maxwell spot centroids in adults without and with dyslexia", Biological Sciences, vol. 284, no. 1865, 25 October 2017 (2017-10-25), page 20171380, XP055490044, ISSN: 0962-8452, DOI: 10.1098/rspb.2017.1380
- Albert Le Floch ET AL: "Left-right asymmetry of the Maxwell spot centroids in adults without and with dyslexia Eleczronic Supplement Material", , 18 October 2017 (2017-10-18), pages 2-3, XP055510300, Retrieved from the Internet: URL:http://rspb.royalsocietypublishing.org /highwire/filestream/77828/field_highwire_ adjunct_files/0/rspb20171380supp1.pdf [retrieved on 2018-09-26]
- La vraie démocratie: "Des physiciens de Rennes ont percé le mystère de la dyslexie", Youtube, 19 October 2017 (2017-10-19), page 2 pp., XP054978716, Retrieved from the Internet: URL:https://www.youtube.com/watch?v=12EoF5 YS8Zg [retrieved on 2018-09-27]

## Description

### 1. Domaine de l'invention.

La présente invention concerne un dispositif d'éclairage. L'invention concerne plus particulièrement un dispositif d'éclairage facilitant la lecture de contenus, notamment textuels, pour des personnes sujettes à la dyslexie.

### 2. Etat de l'art.

La dyslexie est communément définie comme un ensemble de troubles de la lecture qui apparaissent à l'enfance. Il s'agit de troubles spécifiques d'apprentissage dont les causes apparaissent complexes et ont fait et font toujours l'objet de nombreuses études dans des domaines variés.

Il est généralement exclu de considérer que les causes de la dyslexie puissent être uniquement d'ordre sensoriel, social ou psychologique.

Des études menées dans le domaine des neurosciences permettent de penser qu'il pourrait s'agir d'un trouble neurologique spécifique.

Les progrès réalisés dans le domaine de l'imagerie médicale ont pu mettre en évidence le rôle de certaines zones du cerveau dans les processus de lecture et de maîtrise du langage.

Les solutions apportées pour traiter les troubles de la dyslexie s'appuient sur des travaux et activités ludiques selon les difficultés propres à chaque sujet. L'objectif d'un tel accompagnement est d'apporter au sujet objet de troubles une autonomie en matière de lecture. Les méthodes connues sont développées autour de travaux dans des domaines tels que la psychologie, la psychomotricité et l'orthoptie, par exemple.

Récemment, des études ont été conduites, établissant une corrélation entre des particularités propres au mécanisme de la vision et la présence de troubles spécifiques de la dyslexie. La publication "Left-right asymmetry of the Maxwell spot centroids in adults without and with dyslexia (Le Floch A, Ropars G. 2017, Proc. R. Soc. B 284: 20171380, http://dx.doi.org/10.1098/rspb.2017.1380) mentionne le rôle des fovéas, situées dans l'œil humain, dans la construction des images perçues, au niveau cérébral, et le fait que des caractéristiques identiques ou sensiblement identiques pour les deux yeux d'un même sujet entraînent chez lui des dysfonctionnements dans le processus de la vision et du traitement phonologique au niveau cérébral. La transmission d'une image miroir d'un hémisphère à l'autre, par exemple, perturbe sensiblement le processus de lecture d'éléments graphiques ou de contenus textuels chez des sujets présentant des troubles caractéristiques de la dyslexie. Des troubles liés à une instabilité de la fixation et/ou une instabilité posturologique, ou encore des défauts de convergence binoculaire liés aux muscles oculomoteurs, peuvent également entraîner des encombrements visuels comme l'effet miroir.

### 3. Résumé de l'invention.

Selon l'invention, il est proposé un dispositif selon la revendication 1 et un procédé selon la revendication 12.

L'invention permet d'améliorer au moins certains des inconvénients de l'art antérieur en proposant un dispositif d'éclairage adapté à faciliter la lecture de contenus, tels que des contenus graphiques, sur tout type de supports, et notamment sur un support papier. Le dispositif proposé opère des périodes d'activation et de désactivation ou suppression successives d'un faisceau lumineux dans le spectre de la lumière visible. Le faisceau lumineux est ainsi activé et désactivé (supprimé) périodiquement à partir du dispositif d'éclairage selon des cycles successifs opérés à une fréquence prédéterminée comprise dans un intervalle de valeurs allant de 60 à 90 Hz, grâce à un système accordable fonctionnant alors en dispositif anti-encombrement visuel. Ce système met à profit les mécanismes hébbiens dans les neurones du cortex. Les périodes d'activation successives ont chacune une durée comprise dans un intervalle de valeurs allant de 15 à 30% de la durée totale des cycles opérés.

L'invention concerne plus particulièrement un dispositif d'éclairage selon la revendication 1 comprenant une unité de contrôle et un module d'éclairage adapté à la génération d'un faisceau lumineux dans un spectre de lumière visible, caractérisé en ce que ledit dispositif d'éclairage est configuré pour activer et désactiver périodiquement ledit faisceau d'éclairage selon des cycles successifs opérés à une fréquence Fd prédéterminée, chaque cycle ayant une durée T et comprenant une période d'activation du faisceau lumineux de durée T1 précédée ou suivie d'une période de désactivation du faisceau lumineux de durée T2, en ce que la fréquence Fd est comprise dans un intervalle de valeurs allant de 60 à 90 Hz, et en ce que la durée T1 des périodes d'activation du faisceau lumineux est comprise dans un intervalle de valeurs allant de 15 à 30% de la durée T des cycles.

Selon l'invention, il est entendu par « désactivation » du faisceau lumineux, une absence totale d'émission du faisceau lumineux ou encore une émission de puissance bien inférieure au niveau d'émission de la phase d'activation, de sorte que la différence de perception qui en découle induise des effets similaires, sur un sujet dyslexique, aux effets découverts du fait de l'absence de faisceaux selon les cycles précédemment décrits. La désactivation du faisceau lumineux peut donc être totale (le faisceau lumineux est totalement absent après désactivation) ou partielle.

Selon un mode de réalisation de l'invention, la fréquence Fd prédéterminée du faisceau lumineux est définie par un utilisateur du dispositif d'éclairage.

Selon un mode de réalisation de l'invention, la fréquence prédéterminée du faisceau est sélectionnée de façon discrète, c'est-à-dire parmi une pluralité de fréquences prédéterminées dans l'intervalle de fréquences susmentionné.

Selon un mode de réalisation de l'invention, la fréquence Fd varie dans le temps, afin de faciliter plus encore, dans certains cas, l'effacement de l'encombrement visuel et la stabilité binoculaire.

Selon un mode de réalisation de l'invention, la fréquence Fd varie en croissant par pas successifs jusqu'à une valeur maximale selon une première vitesse, dite vitesse de croissance, puis varie en décroissant par pas successifs jusqu'à une valeur minimale selon une seconde vitesse, dite vitesse de décroissance, les variations croissantes et décroissantes se répétant itérativement dans le temps.

Selon un mode de réalisation de l'invention, ladite vitesse de décroissance et égale à ladite vitesse de croissance.

Selon un mode de réalisation de l'invention, lesdits pas successifs sont de durées égales.

Selon un mode de réalisation de l'invention, lesdits pas successifs varient en durée de sorte que la valeur de ladite fréquence Fd évolue selon une forme d'onde en triangle, en scie, ou en sinusoïde entre ladite valeur maximale et ladite valeur minimale.

Selon un mode de réalisation de l'invention, la durée T1 des périodes d'activation du faisceau lumineux varie dans le temps.

Selon un mode de réalisation de l'invention, le faisceau réalisé dans un spectre de lumière visible est généré à partir d'un ou plusieurs éléments de type LED.

Avantageusement, l'utilisation d'une gamme de fréquences à partir de 60 Hz permet de s'affranchir des effets de clignotements perceptibles par l'œil de l'être humain, la limite de perception du clignotement par l'œil se situant aux alentours de 60 Hz, pour l'être humain (hors considération, des espèces animales et des insectes).

Avantageusement, l'alternance de périodes d'activation et de désactivation (ou suppression, ou encore atténuation sensible, ou encore inhibition) du faisceau lumineux, établi dans le spectre de la lumière visible et appliqué à (orienté vers) un support, permet une "focalisation" du cerveau d'un sujet regardant ce support éclairé, sur une image représentative du contenu représenté sur le support observé, puis, une disparition ou quasi-disparition de cette même image de la vue du sujet avant qu'elle ne soit transmise sous forme d'image miroir entre un hémisphère cérébral et l'autre hémisphère cérébral de ce sujet regardant ce support éclairé. Le délai requis pour que le cerveau transmette une image, perçue par l'œil, entre un hémisphère et l'autre hémisphère, sous forme d'image miroir pour ce dernier, est de l'ordre de 10 ms.

Ainsi, le cerveau privilégie l'image transmise par rapport à son image miroir, et la confusion existante chez le sujet qui présente une forte similarité des caractéristiques de ses deux fovéas, est moindre ou sensiblement diminuée pour la lecture du contenu (graphique et/ou textuel) représenté sur le support, notamment lorsque ce contenu est représentatif d'un ou plusieurs contenus textuels.

Avantageusement, l'utilisation d'un commutateur configuré pour obtenir sélectivement un faisceau lumineux permanent (éclairage ordinaire) et un faisceau lumineux non permanent (éclairage selon l'invention) permet à un utilisateur sujet à des troubles dyslexiques de comparer ses performances usuelles en lecture (obtenues avec un éclairage ordinaire) à ses performances obtenues sous contrôle électronique du faisceau lumineux selon l'invention, après optimisation éventuelle de ses propres paramètres (paramètres de fréquence et de rapport cyclique permettant à l'utilisateur d'obtenir un meilleur confort de lecture).

### 4. Liste des figures.

L'invention sera mieux comprise, et d'autres particularités et avantages apparaîtront à la lecture de la description qui va suivre, la description faisant référence aux dessins annexés parmi lesquels :
- la **figure 1** est un schéma de représentation d'un signal EL de commande (contrôle) d'un module d'éclairage selon un mode de réalisation particulier et non limitatif de l'invention.
- la **figure 2** est une représentation structurelle de l'architecture d'un dispositif d'éclairage LIGHT selon un mode de réalisation particulier et non-limitatif de l'invention.

### 5. Description détaillée de modes de réalisation de l'invention.

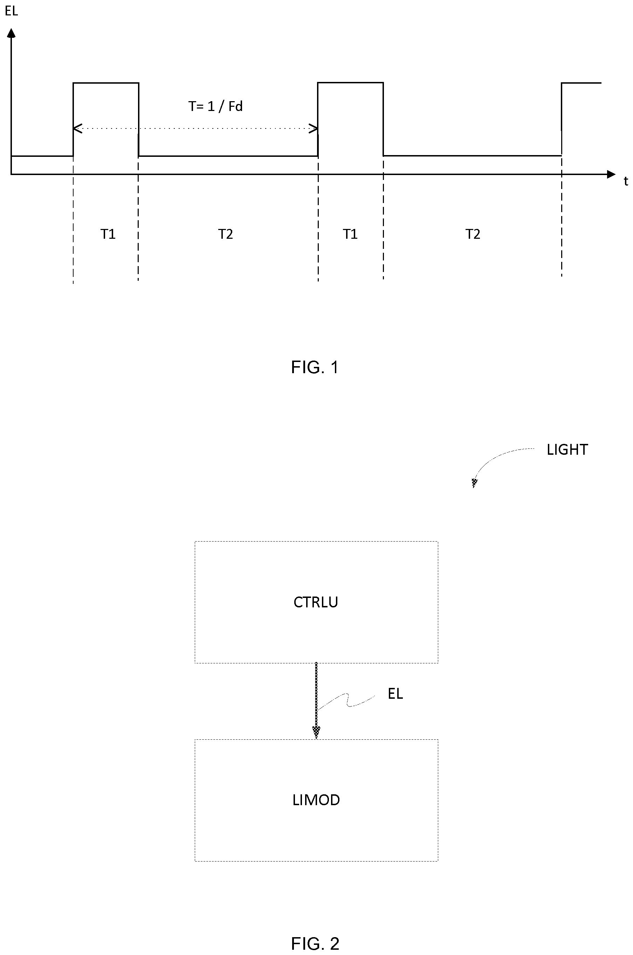

Sur la **figure 2****,** les modules représentés sont des unités fonctionnelles, qui correspondent ou non à des unités physiquement distinguables. Par exemple, ces modules ou certains d'entre eux sont regroupés dans un unique composant. *A contrario,* selon d'autres modes de réalisation, certains modules sont composés d'entités physiques séparées.

La **figure 1** est une représentation temporelle d'un signal EL de commande d'activation et de désactivation (suppression) d'un faisceau d'éclairage dans le spectre de la lumière visible, dans un dispositif d'éclairage LIGHT, selon un mode de réalisation particulier et non limitatif de l'invention. Le signal EL varie selon le temps t et prend périodiquement deux états successifs. Selon le mode de réalisation préféré, une assertion du signal EL à l'état haut contrôle une activation du faisceau d'éclairage du dispositif d'éclairage LIGHT susceptible d'éclairer ainsi, de manière discontinue un contenu graphique et/ou textuel sur un support quelconque, tel qu'un livre, par exemple. Le signal EL désactive (supprime ou inhibe) le faisceau lumineux du module d'éclairage lorsqu'il est positionné à l'état bas. Le signal EL est un signal périodique de fréquence prédéterminé Fd tel que Fd = 1/(T1+T2). T1 est la période d'activation du faisceau lumineux dans le spectre de la lumière visible, soit la période d'éclairage d'un support portant un motif représentant un contenu graphique et/ou textuel vers lequel le faisceau est orienté. T2 est la période, dite période de désactivation, durant laquelle le faisceau est supprimé ou rendu inactif, ou en d'autres termes la période pendant laquelle le contenu graphique et/ou textuel sur un support vers lequel le faisceau est orienté n'est plus éclairé par le faisceau. Les termes "contenu graphique" sont à interpréter ici comme n'importe quel contenu représenté sur un support quelconque, notamment au format papier mais pas seulement, et constitué d'éléments élémentaires tels que, à titre d'exemple des points ou pixels juxtaposés, de sorte que le contenu représente des éléments de formes variées et notamment un ou plusieurs contenus textuels construits à partir de signes ou de symboles d'un ou plusieurs alphabets.

Ainsi un contenu textuel apposé sur un support correspond ici à un contenu interprétable dans un ou plusieurs langages, susceptible d'être lu et interprété par un sujet, utilisateur du dispositif d'éclairage, positionné de sorte à regarder le support ainsi éclairé pour une opération de lecture ou de visualisation. Un tel dispositif est, à titre d'exemple, une lampe de bureau, une lampe torche, une lampe frontale, un stylo-lampe, une lampe de poche, une lampe de téléphone, une lampe de smartphone, une montre-lampe, une lampe de porte clef, un plafonnier, un lampadaire, une ampoule, un tube d'éclairage, un projecteur, un projecteur d'automobile, un rétroprojecteur, un revêtement mural lumineux, un tapis lumineux, un bracelet lumineux, un cadre lumineux, une mini-lampe à positionner sur un livre par le biais d'un système d'attache, un dispositif configuré pour l'éclairage intérieur (d'un local) ou encore un dispositif configuré pour un éclairage extérieur, urbain ou non. Cette liste d'exemples n'étant pas exhaustive. Selon le mode de réalisation préféré de l'invention, le rapport cyclique T1/(T1+T2) entre les périodes d'activation et de désactivation (ou inhibition) du faisceau lumineux, respectivement de durées T1 et T2, a une valeur comprise entre 15% et 30% du cycle, et la fréquence Fd de variation du signal EL est comprise entre 60 Hz et 90 Hz.

De façon préférée, la fréquence du signal est égale à 70 Hz ou 84 Hz et le rapport cyclique T1 / (T1+T2) est égal à 20%.

Avantageusement, le signal de contrôle EL peut être aisément forcé de façon prolongée dans son état associé à une activation du faisceau lumineux, ce qui correspond à un débrayage de la méthode d'éclairage mise en œuvre dans le dispositif LIGHT selon l'invention. Il serait ainsi possible de ne pas mettre en œuvre la méthode de contrôle d'un faisceau lumineux, dans un dispositif d'éclairage selon l'invention, dans le cas où, pour un sujet non dyslexique, une gêne visuelle apparaitrait du fait de la discontinuité d'activation du faisceau d'éclairage.

Selon l'invention, il est possible d'affiner le réglage de la fréquence Fd dans l'intervalle de valeurs décrit dans le but d'adapter la période T à la sensibilité d'un utilisateur du dispositif d'éclairage LIGHT, dans la gamme de fréquences indiquée. En effet, chaque individu possède une sensibilité propre en termes de vision et perçoit plus ou moins des variations de fréquence d'un faisceau lumineux. Ainsi, un réglage fin peut être rendu accessible à l'utilisateur par l'intermédiaire d'un bouton de réglage, d'un curseur, implémenté matériellement ou via une interface utilisateur quelconque (éléments graphiques d'un menu sur un écran de contrôle, par exemple).

La **figure 2** est une représentation structurelle d'un dispositif d'éclairage LIGHT selon un mode de réalisation particulier et non-limitatif de l'invention. Cette figure représente l'architecture globale du dispositif d'éclairage LIGHT, encore communément appelé « lampe ». Le dispositif LIGHT comprend deux modules principaux qui sont une unité de contrôle CTRLU et un module d'éclairage LIMOD. L'unité de contrôle CTRLU est le cœur du système en termes de contrôle et comprend un circuit bistable (ou hacheur) classique, adapté à la génération du signal EL. Le circuit de hachage bistable de l'unité de contrôle CTRLU délivre le signal EL caractérisé par la fréquence Fd et par son rapport cyclique T1/(T1 +T2). Bien évidemment l'unité de contrôle CTRLU comprend tous les éléments usuels mis en œuvre dans une telle architecture, tels que, à titre d'exemples, un ou plusieurs amplificateurs opérationnels, des résistances et capacités, une ou plusieurs diodes, une alimentation, un circuit de remise à zéro, un circuit de supervision d'alimentation, une interface de puissance, un amplificateur de courant, la liste de ces éléments n'est pas exhaustive. Les détails architecturaux de l'unité de contrôle CTRLU ne sont pas décrits plus encore dans la mesure où ceux-ci ne sont pas utiles à la compréhension de l'invention. Selon un mode de réalisation de l'invention, le module CTRLU comprend un circuit bistable construit autour d'un amplificateur opérationnel, couplé à un circuit d'amplification de courant. Avantageusement, l'utilisation d'un amplificateur de courant permet d'obtenir une énergie moyenne suffisante à un éclairage correct bien que le faisceau lumineux ainsi généré soit discontinu. Le module d'éclairage LIMOD est un module d'éclairage adapté à la génération d'un faisceau lumineux dans le spectre de la lumière visible, ou sensiblement plus large. Avantageusement, le faisceau peut être plus ou moins focalisé pour être alors configuré pour l'éclairage d'une surface plus ou moins grande. Une telle focalisation peut être réalisée par l'utilisation d'éléments optiques (lentilles) ou encore d'éléments mécaniques (diaphragmes), ou les deux à la fois.

Avantageusement, le faisceau lumineux est réalisé à partir d'un ou plusieurs objets électroluminescents de type LED, de l'acronyme anglais « Light-Emitting Diode » et qui signifie « diode à émission de lumière ». Bien évidemment, le faisceau lumineux peut être réalisé à partir d'autres éléments lumineux, dès lors que la désactivation du faisceau peut-être suffisamment rapide pour respecter les cycles comprenant des périodes d'éclairage et d'inhibition du faisceau (périodes de non-éclairage).

Le terme « inhibition du faisceau » doit être interprété ici comme une disparition totale du faisceau ou un amoindrissement conséquent du niveau d'éclairage produit par le faisceau.

C'est la capacité à activer et inhiber successivement l'éclairage d'un support qui présente un ou plusieurs contenus graphiques et/ou textuels, sous contrôle du module bistable de l'unité CTRLU, qui permet avantageusement au cerveau d'un sujet de privilégier une image plutôt que son image miroir, perçue à partir du support lorsque ce dernier est éclairé par le dispositif d'éclairage LIGHT selon l'invention. Avantageusement, cela permet d'aider conséquemment la lecture et le déchiffrage de contenus textuels, chez un sujet présentant des troubles dyslexiques.

Avantageusement, l'unité de contrôle CTRLU comprend en sortie un signal EL d'activation (ou d'extinction / inhibition) du faisceau d'éclairage, connecté en entrée du module d'éclairage LIMOD.

En d'autres termes, les variations du signal de contrôle du faisceau d'éclairage EL, opérées par l'unité de contrôle CTRLU comprenant un circuit bistable, à une fréquence Fd, permettent d'agir sur l'éclairage d'un contenu graphique représenté sur un support quelconque, de sorte que ce contenu graphique soit successivement éclairé puis moins (ou plus du tout) éclairé, périodiquement, sur le support, selon des cycles successifs de longueur totale T opérés à une fréquence Fd prédéterminée. Selon l'invention, les périodes d'éclairage successives T1 ont chacune une durée comprise dans un intervalle de valeurs allant de 15 à 30% de la durée T desdits cycles. La fréquence Fd des cycles (comprenant chacun une période d'activation du faisceau et une période de désactivation du faisceau) est comprise entre 60 et 90 Hz.

Selon un mode de réalisation, la fréquence Fd est fixe.

Selon un autre mode de réalisation, la fréquence Fd varie dans le temps.

Selon un mode de réalisation particulier, la fréquence Fd varie en croissant par pas successifs jusqu'à une valeur maximale selon une première vitesse, dite vitesse de croissance, puis varie en décroissant par pas successifs jusqu'à une valeur minimale selon une seconde vitesse, dite vitesse de décroissance, les variations croissantes et décroissantes se répétant itérativement dans le temps.

Selon un mode de réalisation particulier, la vitesse de décroissance et égale à la vitesse de croissance.

Selon un mode de réalisation particulier, les pas successifs sont de durées égales.

Selon un autre mode de réalisation particulier, les pas successifs varient en durée de sorte que la valeur de ladite fréquence Fd évolue selon une forme d'onde en triangle, en scie, ou en sinusoïde entre ladite valeur maximale et ladite valeur minimale.

Avantageusement la durée T1 des périodes d'activation varie dans le temps en évoluant de façon continue ou discontinue entre des valeurs bornes allant de 15 à 30% de la durée T des cycles. Il est entendu ici par de « façon continue » une évolution par incrément des pas successifs de durées égales.

Le phénomène de wobulation ainsi créé et appliqué à la fréquence Fd prédéterminée (variation de la fréquence Fd) permet de balayer un grand nombre de fréquences entre 60 Hz et 90 Hz, dont certaines seront plus efficaces pour l'aide à la lecture. Ces fréquences plus efficaces varient selon le sujet dyslexique. En balayant toutes les fréquences entre 60 et 90 Hz, le dispositif de l'invention ne nécessite aucun réglage préalable et devient efficace pour un grand nombre d'utilisateurs. Ce phénomène de wobulation permet ainsi et dans certains cas, de réduire plus encore des ennuis liés à des troubles dyslexiques.

Un même avantage découle des variations de la durée T1 des périodes d'activation du faisceau lumineux.

Selon un autre mode de réalisation de l'invention, le dispositif d'éclairage LIGHT est configuré pour générer un premier faisceau lumineux continu pendant toute sa durée d'utilisation et un dispositif mobile ou de filtrage, piloté ou mû, masque ou inhibe périodiquement le faisceau lumineux de sorte qu'un second faisceau soit généré avec des caractéristiques de fréquence et de rapport cycliques tels que ceux précédemment décrits (Fd comprise en 60 Hz et 90 Hz et T1/(T1+T2) entre 15 et 30 %. A titre d'exemples non-limitatifs, selon ce mode de réalisation, un premier faisceau peut être interrompu cycliquement par un élément rotatif présentant des surfaces pleines et des surfaces vides, ou encore des surfaces opaques et des surfaces translucides, adaptées, par leur rotation, à générer le second faisceau, dont l'effet est alors le même que celui décrit dans le premier mode de réalisation décrit précédemment.

L'invention ne se limite pas aux seuls modes de réalisation décrits ciavant, mais s'applique à tout dispositif d'éclairage d'un contenu graphique et/ou textuel sur un support quelconque, mettant en œuvre des opérations successives d'activation d'un faisceau d'éclairage et d'inhibition de ce même faisceau, périodiquement, selon des cycles successifs opérés à une fréquence Fd prédéterminée entre 60 Hz et 90 Hz telle que les périodes d'éclairage T1 successives ont chacune une durée comprise dans un intervalle de valeurs allant de 15 à 30% de la durée T cycles opérés.

## Revendications

1. Dispositif d'éclairage (LIGHT) pour faciliter la lecture d'un contenu graphique et/ou textuel sur un support quelconque par des sujets dyslexiques, ledit dispositif comprenant une unité de contrôle (CTRLU) et un module d'éclairage (LIMOD) adapté à la génération d'un faisceau lumineux dans un spectre de lumière visible, ledit dispositif d'éclairage (LIGHT) étant configuré pour activer et désactiver périodiquement ledit faisceau lumineux selon des cycles successifs opérés à une fréquence prédéterminée Fd qui est une réciproque de la somme d'une période d'activation du faisceau lumineux et d'une période de désactivation du faisceau lumineux, chaque cycle ayant une durée T et comprenant une période d'activation du faisceau lumineux de durée T1 suivie ou précédée d'une période de désactivation du faisceau lumineux de durée T2, Fd étant tel que Fd = 1/(T1 +T2), la fréquence prédéterminée Fd étant comprise dans un intervalle de valeurs allant de 60 à 90 Hz, et la durée T1 des périodes d'activation du faisceau lumineux étant comprise dans un intervalle de valeurs allant de 15 à 30% de la durée T des cycles ledit dispositif étant **caractérisé en ce qu'**il comprend un bouton de réglage ou un curseur, implémenté matériellement ou via une interface utilisateur, pour affiner la fréquence prédéterminée Fd dans l'intervalle de valeurs allant de 60 à 90 Hz.

2. Dispositif d'éclairage selon la revendication 1, dans lequel ladite fréquence prédéterminée Fd est égale à 70 Hz ou 84 Hz et le rapport cyclique T1 / (T1+T2) est égal à 20%.

3. Dispositif d'éclairage selon la revendication 1 ou 2, configuré pour varier ladite fréquence prédéterminée Fd dans le temps.

4. Dispositif d'éclairage selon la revendication 3, dans lequel ladite fréquence prédéterminée Fd est dite de wobulation, mettant à profit les mécanismes hébbiens des neurones du cortex et le dispositif d'éclairage est configuré pour varier la fréquence prédéterminée Fd en croissant par pas successifs jusqu'à une valeur maximale selon une première vitesse, dite vitesse de croissance, puis varier en décroissant par pas successifs jusqu'à une valeur minimale selon une seconde vitesse, dite vitesse de décroissance, les variations croissantes et décroissantes se répétant itérativement dans le temps.

5. Dispositif d'éclairage selon la revendication 4, dans lequel ladite vitesse de décroissance est égale à ladite vitesse de croissance.

6. Dispositif d'éclairage selon la revendication 5, dans lequel lesdits pas successifs sont de durées égales.

7. Dispositif d'éclairage selon la revendication 5, configuré pour varier lesdits pas successifs en durée de sorte que la valeur de ladite fréquence prédéterminée Fd évolue selon une forme d'onde en triangle, en scie, ou en sinusoïde entre ladite valeur maximale et ladite valeur minimale.

8. Dispositif d'éclairage selon l'une quelconque des revendications précédentes, configuré pour varier la durée T1 desdites périodes d'activation du faisceau lumineux dans le temps.

9. Dispositif d'éclairage selon l'une quelconque des revendications précédentes, dans lequel ledit faisceau lumineux est généré à partir d'un ou plusieurs éléments de type LED faisant partis du dispositif d'éclairage.

10. Dispositif d'éclairage selon l'une quelconque des revendications précédentes, **caractérisé en ce qu'**il est d'un type compris dans la liste : une lampe de bureau, une lampe torche, une lampe frontale, un stylo-lampe, une lampe de poche, une lampe de téléphone, une lampe de smartphone, une montre-lampe, une lampe de porte clef, un plafonnier, un lampadaire, une ampoule, un tube d'éclairage, un projecteur, un projecteur d'automobile, un rétroprojecteur, un revêtement mural lumineux, un tapis lumineux, un bracelet lumineux, un cadre lumineux, une mini-lampe à positionner sur un livre, un dispositif configuré pour l'éclairage intérieur ou encore un dispositif configuré pour un éclairage extérieur, urbain ou non.

11. Dispositif d'éclairage selon l'une quelconque des revendications précédentes, **caractérisé en ce qu'**il s'agit d'une lampe de bureau ou d'une mini-lampe à positionner sur un livre.

12. Procédé pour faciliter la lecture d'un contenu graphique et/ou textuel sur un support quelconque par un sujet dyslexique, **caractérisé en ce qu'**il met en œuvre un dispositif d'éclairage tel que défini selon l'une quelconque des revendications précédentes, que l'on active et désactive périodiquement selon des cycles successifs opérés à une fréquence prédéterminée Fd, chaque cycle ayant une durée T et comprenant une période d'activation du faisceau lumineux de durée T1 précédée ou suivie d'une période de désactivation du faisceau lumineux de durée T2,
- la fréquence prédéterminée Fd étant définie par l'équation Fd = 1/(T1+T2)
- Fd étant comprise dans un intervalle de valeurs allant de 60 à 90 Hz, et
- la durée T1 des périodes d'activation du faisceau lumineux étant comprise dans un intervalle de valeurs allant de 15 à 30% de la durée T des cycles ladite fréquence prédéterminée Fd étant définie par un utilisateur dudit dispositif d'éclairage par l'intermédiaire d'un bouton de réglage ou un curseur, implémenté matériellement ou via une interface utilisateur, pour affiner la fréquence prédéterminée Fd dans l'intervalle de valeurs allant de 60 à 90 Hz.

## Patentansprüche

1. Beleuchtungsvorrichtung (LIGHT) zur Erleichterung des Lesens eines Grafik- und/oder Textinhalts auf einem beliebigen Träger durch Legastheniker, wobei die Vorrichtung eine Steuereinheit (CTRLU) und ein Beleuchtungsmodul (LIMOD), das zur Erzeugung eines Lichtbündels in einem Spektrum sichtbaren Lichts geeignet ist, umfasst, wobei die Beleuchtungsvorrichtung (LIGHT) dazu ausgebildet ist, das Lichtbündel gemäß aufeinanderfolgenden Zyklen periodisch zu aktivieren und zu desaktivieren, die mit einer vorbestimmten Frequenz Fd erfolgen, die ein Kehrwert der Summe einer Aktivierungsperiode des Lichtbündels und einer Deaktivierungsperiode des Lichtbündels ist, wobei jeder Zyklus eine Dauer T aufweist und eine Aktivierungsperiode des Lichtbündels mit einer Dauer T1 umfasst, der eine Deaktivierungsperiode des Lichtbündels mit einer Dauer T2 folgt oder vorangeht, wobei Fd so ist, dass Fd = 1/(T1 +T2), wobei die vorbestimmte Frequenz Fd in einem Werteintervall von 60 bis 90 Hz liegt und die Dauer T1 der Aktivierungsperioden des Lichtbündels in einem Werteintervall von 15 bis 30 % der Dauer T der Zyklen liegt, wobei die Vorrichtung **dadurch gekennzeichnet ist, dass** sie einen Regelknopf oder einen Cursor umfasst, der materiell oder über eine Benutzerschnittstelle implementiert ist, um die vorbestimmte Frequenz Fd im Werteintervall von 60 bis 90 Hz zu verfeinern.

2. Beleuchtungsvorrichtung nach Anspruch 1, wobei die vorbestimmte Frequenz Fd gleich 70 Hz oder 84 Hz und das Tastverhältnis T1 / (T1+T2) gleich 20 % ist.

3. Beleuchtungsvorrichtung nach Anspruch 1 oder 2, die dazu ausgebildet ist, die vorbestimmte Frequenz Fd zeitlich zu variieren.

4. Beleuchtungsvorrichtung nach Anspruch 3, wobei die vorbestimmte Frequenz Fd eine Wobbelfrequenz ist, die die hebbschen Mechanismen der Neuronen der Kortex ausnutzt, und die Beleuchtungsvorrichtung dazu ausgebildet ist, die vorbestimmte Frequenz Fd durch Steigerung in sukzessiven Schritten bis zu einem Maximalwert mit einer ersten Geschwindigkeit, der Steigerungsgeschwindigkeit, zu variieren, dann durch Absenkung in sukzessiven Schritten bis zu einem Minimalwert mit einer zweiten Geschwindigkeit, der Absenkungsgeschwindigkeit, zu variieren, wobei sich die Steigerungs- und die Absenkungsvariationen zeitlich iterativ wiederholen.

5. Beleuchtungsvorrichtung nach Anspruch 4, wobei die Absenkungsgeschwindigkeit gleich der Steigerungsgeschwindigkeit ist.

6. Beleuchtungsvorrichtung nach Anspruch 5, wobei die sukzessiven Schritte die gleiche Dauer aufweisen.

7. Beleuchtungsvorrichtung nach Anspruch 5, die dazu ausgebildet ist, die sukzessiven Schritte in der Dauer zu variieren, so dass sich der Wert der vorbestimmten Frequenz Fd in einer Dreiecks-, Sägezahn- oder Sinuswellenform zwischen dem Maximalwert und dem Minimalwert bewegt.

8. Beleuchtungsvorrichtung nach einem der vorhergehenden Ansprüche, die dazu ausgebildet ist, die Dauer T1 der Aktivierungsperioden des Lichtbündels zeitlich zu variieren.

9. Beleuchtungsvorrichtung nach einem der vorhergehenden Ansprüche, wobei das Lichtbündel von einem oder mehreren LED-Elementen erzeugt wird, die zur Beleuchtungsvorrichtung gehören.

10. Beleuchtungsvorrichtung nach einem der vorhergehenden Ansprüche, **dadurch gekennzeichnet, dass** sie von einem Typ ist, der in der folgenden Liste enthalten ist: eine Schreibtischlampe, eine Handlampe, eine Stirnlampe, eine Stiftlampe, eine Taschenlampe, eine Telefonlampe, eine Smartphone-Lampe, eine Lampenuhr, eine Schlüsselanhängerlampe, eine Deckenleuchte, eine Stehlampe, eine Glühbirne, eine Leuchtröhre, ein Scheinwerfer, ein Autoscheinwerfer, einen Overheadprojektor, eine beleuchtete Wandverkleidung, einen Leuchtteppich, ein Leuchtarmband, einen Leuchtrahmen, eine Minilampe zur Positionierung auf einem Buch, eine für die Innenbeleuchtung ausgebildete Vorrichtung oder auch eine für die Außenbeleuchtung in der Stadt oder außerhalb ausgebildete Vorrichtung.

11. Beleuchtungsvorrichtung nach einem der vorhergehenden Ansprüche, **dadurch gekennzeichnet, dass** es sich um eine Schreibtischlampe oder eine Minilampe zur Positionierung auf einem Buch handelt.

12. Verfahren zur Erleichterung des Lesens eines Grafik- und/oder Textinhalts auf einem beliebigen Träger durch einen Legastheniker, **dadurch gekennzeichnet, dass** dabei eine Beleuchtungsvorrichtung nach einem der vorhergehenden Ansprüche verwendet wird, die gemäß aufeinanderfolgenden Zyklen periodisch aktiviert und deaktiviert wird, die mit einer vorbestimmten Frequenz Fd erfolgen, wobei jeder Zyklus eine Dauer T aufweist und eine Aktivierungsperiode des Lichtbündels mit einer Dauer T1 umfasst, der eine Deaktivierungsperiode des Lichtbündels mit einer Dauer T2 folgt oder vorangeht,
- wobei die vorbestimmte Frequenz Fd durch die Gleichung Fd = 1/(T1+T2) definiert wird
- wobei Fd in einem Werteintervall von 60 bis 90 Hz liegt, und
- wobei die Dauer T1 der Aktivierungsperioden des Lichtbündels in einem Werteintervall von 15 bis 30 % der Dauer T der Zyklen liegt, wobei die vorbestimmte Frequenz Fd von einem Benutzer der Beleuchtungsvorrichtung mittels eines Regelknopfes oder eines Cursors festgelegt wird, der materiell oder über eine Benutzerschnittstelle implementiert ist, um die vorbestimmte Frequenz Fd im Werteintervall von 60 bis 90 Hz zu verfeinern.

## Claims

1. Lighting device (LIGHT) for facilitating the reading of graphic and/or textual content on any given support by dyslexic subjects, said device comprising a control unit (CTRLU) and a lighting module (LIMOD) adapted to the generation of a light beam in a spectrum of visible light, said lighting device (LIGHT) being configured to periodically activate and deactivate said light beam according to successive cycles carried out at a predetermined frequency Fd that is a reciprocal of the sum of a period of activation of the light beam and of a period of deactivation of the light beam, each cycle having a duration T and comprising a period of activation of the light beam having a duration T1 preceded or followed by a period of deactivation of the light beam having a duration T2, Fd being such that Fd = 1/(T1+T2), the predetermined frequency Fd being comprised in an interval of values ranging from 60 to 90 Hz, and in that the duration T1 of the periods of activation of the light beam is comprised in an interval of values ranging from 15 to 30% of the duration T of the cycles, said device being **characterised in that** it comprises an adjustment knob or a slider, implemented in hardware or via a user interface, for refining the predetermined frequency Fd in the range of values from 60 to 90 Hz.

2. Lighting device according to claim 1, wherein said predetermined frequency Fd is equal to 70 Hz or 84 Hz and the duty cycle T1 / (T1+T2) is equal to 20%.

3. Lighting device according to claim 1 or 2, configured to vary said predetermined frequency Fd over time.

4. Lighting device according to claim 3, wherein said predetermined frequency Fd is called wobulation frequency, takes advantage of the Hebbian mechanisms of the neurons of the cortex and the lighting device is configured to vary the predetermined frequency Fd by increasing by successive steps up to a maximum value according to a first speed, called increasing speed, then to vary by decreasing by successive steps down to a minimum value according to a second speed, called decreasing speed, the increasing and decreasing variations repeating iteratively over time.

5. Lighting device according to claim 4, wherein said decreasing speed is equal to said increasing speed.

6. Lighting device according to claim 5, wherein said successive steps have equal durations.

7. Lighting device according to claim 5, configured to vary in duration said successive steps so that the value of said predetermined frequency Fd changes according to a waveform in the shape of a triangle, a saw, or a sinusoid between said maximum value and said minimum value.

8. Lighting device according to any of the previous claims, configured to vary over time the duration T1 of said periods of activation of the light beam.

9. Lighting device according to any of the previous claims, wherein said light beam is generated using one or more elements of the LED type being part of the lighting device.

10. Lighting device according to any of the previous claims, **characterised in that** it is of a type comprised in the list: a table lamp, a torch, a headlamp, a pen light, a pocket lamp, a telephone lamp, a smartphone lamp, a torch watch, a keyring lamp, a ceiling light, a standard lamp, a lightbulb, a lighting tube, a projector, a motor vehicle projector, an overhead projector, a luminous wall coating, a light-up rug, a light-up bracelet, a light-up frame, a mini-lamp to be positioned on a book, a device configured for interior lighting or a device configured for exterior lighting, urban or not.

11. Lighting device according to any of the previous claims, **characterised in that** it is a table lamp or a mini-lamp to be positioned on a book.

12. Method for facilitating the reading of graphic and/or textual content on any given support by a dyslexic subject, **characterised in that** it implements a lighting device as defined according to any of the previous claims, that is periodically activated and deactivated according to successive cycles carried out at a predetermined frequency Fd, each cycle having a duration T and comprising a period of activation of the light beam having a duration T1 preceded or followed by a period of deactivation of the light beam having a duration T2,
- the predetermined frequency Fd being defined by the equation Fd = 1/(T1+T2),
- Fd being comprised in an interval of values ranging from 60 to 90 Hz, and
- the duration T1 of the periods of activation of the light beam being comprised in an interval of values ranging from 15 to 30% of the duration T of the cycles, said predetermined frequency Fd being defined by a user of said lighting device via an adjustment knob or a slider, implemented in hardware or via a user interface, to refine the predetermined frequency Fd in the range of values from 60 to 90 Hz.
